# EUROPEAN PATENT SPECIFICATION

(11) **EP 2 905 328 B1**
(45) Date of publication and mention of the grant of the patent: **12.08.2020**
(21) Application number: 14460133.3
(22) Date of filing: 23.12.2014
(51) Int. Cl.: C12M 1/107, C12M 1/00, C12M 1/26, C12M 1/34

(54) **METHOD AND SYSTEM FOR PROCESSING ORGANIC MATTER BY ANAEROBIC DIGESTION**
VERFAHREN UND SYSTEM ZUR VERARBEITUNG VON ORGANISCHEM MATERIAL DURCH ANAEROBE VERGÄRUNG
PROCÉDÉ ET SYSTÈME DE TRAITEMENT DE MATIÈRE ORGANIQUE PAR DIGESTION ANAÉROBIE

(30) Priority: 24.12.2013 PL 40670513
(43) Date of publication of application: 12.08.2015
(73) Proprietor: SYGMA Sp. z o.o., 54-211 Wroclaw (PL); Lukaszewicz, Marcin Ziemowit, 51-351 Wroclaw (PL); Sygit, Maciej, 54-211 Wroclaw (PL)
(72) Inventor: Lukaszewicz, Marcin Ziemowit, 51-351 Wroclaw (PL); Sygit, Maciej, 54-211 Wroclaw (PL)
(74) Representative: Winohradnik, J. Halina

(56) References cited:
- EP-A2- 0 036 065
- GB-A- 2 158 840
- US-A1- 2013 324 406

## Description

The subject matter of the invention is a method of processing organic matter by anaerobic digestion and a system for processing organic matter by anaerobic digestion as defined in the appended claims.

The process of anaerobic decomposition of organic carbon containing matter that has been known and used so far, consists in microbiological conversion of organic matter into biogas that contains mostly methane and carbon dioxide along with traces of other gases such as hydrogen sulphide, hydrogen, oxygen, ammonia or nitrogen. According to the methods in use so far, all stages of the methane digestion take place as a rule in one vessel, where organic materials are hydrolysed, converted into organic acids, and through acetates - into methane by active anaerobic microorganisms.

Each process stage occurs with participation of different bacteria species, in one and the same vessel, and so decomposition of materials containing organic carbon needs long time, necessary for microorganisms to perform complex biochemical transformations. In order to ensure suitable efficiency of all process stages proceeding in a single vessel, large-size bioreactors are required, and for acceleration of the processes, the solution in the application No. WO 2011/005079 (A) under PCT procedure, provides for application of elevated pressure in the sewage treatment vessel. According to this solution, the compressed gas energy is utilised to drive pumps involved in operation of the system. According to the method described in the patent application No. US 2007/0224669 (A), the anaerobic digestion microorganisms are employed to generate pressure in a bioreactor and to increase methane content in the biogas utilised by the power industry.

In the system known from the application No. WO 2008/142007 (A) under PCT procedure, a pre-bioreactor is placed in front of the main one-vessel bioreactor and packed with tubes that afford possibilities for formation of a biofilm consisting of microorganisms, owing to which the pre-hydrolytic reactions have been partially separated from the actual methanogenesis.

An anaerobic gas digester which is the object of the GB 2158840 patent application comprises several tanks connected in series in such a way that a top of a preceding tank is connected to a top of a consecutive tank, and each succeeding tank is connected to the top of the preceding one, alike a bottom of a preceding tank is connected to a bottom of a consecutive tank, thus the tanks form larger groups. The method adopted in this digester is characterised by purely one-way flow of the reaction mixture, and also that the gas pressure is converted to mechanical energy which can be employed to drive pumps.

The US 2013/0324406 A1 document discloses a multiphase method of generation of a product containing a bacterial consortium from an organic material, where the main essential feature of the method is splitting the process into a gradient of micro-environments that are adapted to conditions. The method includes a hydrolysis of organic matter, acetogenesis of the hydrolysed matter, followed by transformation of the obtained output containing hydrogen, carbon dioxide, volatile organic acids and methanogenic precursors to methane and denitrification thereof. In the bioreactor system for implementation of this method, the material is continuously moved from one phase to another in predetermined direction at a specific flow rate, while excluding the reciprocating flow of the reaction mixture.

A method of biogas production from agricultural organic waste was disclosed in the patent application EP0036065. This method consists in that the organic mass is fed continuously, no mixing applied, into reactors that are mutually connected at the bottom, and the biogas is received in the upper part of reactors. According to this solution, the system for biogas production from organic waste comprises at least one pressurised digestion vessel, a gas receiver and a digestate drain pipe at the bottom.

In case of large one-vessel bioreactors, where all process phases proceed concurrently, differentiation of pressure, pH, temperature and substrate composition is not possible.

The object of the present invention is to develop a method that would make a possibility for separation of sequentially proceeding anaerobic digestion processes and for separation of quick-decomposing fraction from the fraction requiring longer digestion time, and thereby to achieve acceleration of the anaerobic digestion.

The method of processing organic matter by anaerobic digestion in conjunction with biogas production consists in that each of the four stages of the process, where hydrolysis of macromolecular compounds to smaller molecules is the first stage, these are converted to organic acids in the second stage, which compounds are processed into acetates, methanol and carbon dioxide in the third stage, and the processing to methane makes the fourth stage, proceeds within individually spontaneously created reaction zones, under elevated hydraulic pressure, changing in pulses from atmospheric pressure to 500 bar, with involvement of microorganisms that are specific for the given processing stage. The said consecutive stages of the process proceed at the temperature ranging from 4°C to 180°C, in bioreactor chambers of length suitable for reactions proceeding therein, possibly connected in series, where the ratio of total length of the bioreactor reaction chambers to the mean diameter of the chambers is 1 to 1000000. The microorganisms specific for the given stage and conditions of running the anaerobic digestion process are preferably in form of a high-surface area biofilm, acting as a catalyst to occurring biochemical conversions, which film is obtained by immobilization of appropriate microorganisms in a bed, each reaction chamber is filled with. In each reaction zone the reaction mixture is subject to mixing, and the reaction products are transferred from one zone to another in form of pulsating reciprocating stream of reaction mixture at the rate from 0.01 m/h to 180 m/ h. At the same time, the undissolved fraction that needs longer time of digestion and is separated from obtained soluble decomposition products, is continuously removed from the bioreactor sedimentation zone to a bioreactor operating in parallel, of extended hydraulic retention time.

According to this method, during the process the produced biogas is received in pulsating way. The received biogas is furthermore stored under pressure that results from the process, which in consequence makes possible to decrease the size of the system. Moreover, the compressed biogas is used for pulsating agitation in the bioreactor..

The separated fraction that requires longer digestion time is subject to breaking up, preferably by mixing with unreacted inorganic molecules, such as sand.

Preferably, each stage of the process is run at selected pH, which ranges between 4.5 - 8.0 for the stage of macromolecular compounds hydrolysis and for the stage of their conversion into organic acids, pH range is 5 - 7.5 in the stage of conversion to acetates, methanol and carbon dioxide, and 6 - 9 in the stage of processing to methane.

Preferably, in each reaction zone controlled is temperature, density, and pressure.

Furthermore, partial sanitation of digestate is achieved through a violent expansion thereof.

The biogas obtained, by the method according to the invention is characterised by higher methane content as compared with biogas obtained by methods where near-atmospheric pressure was applied.

An advantage of this method is that it allows self-control and makes possible to separate processes of anaerobic digestion proceeding in sequential stages as it occurs in a digestive tract, between respective reaction zones.

Dividing the biomass being processed into a quick-decomposing fraction and a fraction requiring longer digestion time makes possible to significantly decrease total volume of bioreactors, necessary for processing organic matter by anaerobic digestion at a particular rate, and owing to elevated pressure, the process running temperature may be reduced.

The system for processing organic matter by anaerobic digestion consists of two processing sections operating under different pressures. The first section, for processing the quick-decomposing fraction, comprises the following components connected to one another consecutively: substrate tank, substrate metering pump, ascending vessel providing reciprocating movement of the substrate through this section and at least one bioreactor. In addition, in the upper part of the bioreactor situated is a separatory zone, connected by a pipeline provided with a digestate valve to the digestate tank, for removing from the bioreactor the digestate which is separate from the biogas, while the biogas is received from the bioreactor separatory zone by a pipeline provided with a gas valve. In the option of several bioreactors connected in series, the separatory zone is situated only in the last bioreactor in the series. In the bottom, preferably of a conic shape, of each bioreactor mounted is an outlet pipeline provided with a valve for removing the sedimenting fraction into the bioreactor placed in the processing section, where required is longer digestion time. The gas valve maintains the pre-set pressure in the section, while the digestate valve provides cyclic sudden pressure changes as a result of pulsating outflow of the digestate.

The second section is designed for processing the fraction that requires longer digestion time, and is fed from the first section. This second section comprises the following components connected to one another consecutively: ascending vessel providing reciprocating movement of the substrate and at least one bioreactor, in the upper part of which situated is a separatory zone, connected by a pipeline provided with a digestate valve to the digestate tank, for removing from the bioreactor the digestate which is separate from the biogas, while the biogas is received from the bioreactor separatory zone by a pipeline provided with a gas valve.

The gas valves, the system is provided with, make possible to maintain higher pressure in the section for processing the quick-decomposing fraction, than the pressure in the section for processing the fraction that requires longer digestion time. The digestate valves and check valves connected to bioreactors give pulsating pressure changes compensated by the ascending vessels, and cause reciprocating movement in the processing sections. Preferably, both sections are connected to the biogas pressure tanks, wherein the biogas is stored under pressure.

Preferably, the bioreactors are filled with a bed, immobilizing microorganisms that are specific for a given stage and conditions of proceeding the anaerobic digestion. Preferably, within the bioreactor reaction chambers placed are elements of filling of high-surface area, designed for development of` a biofilm acting as a catalyst in biochemical reactions.

Preferably, the section for processing the quick-decomposing fraction comprises from 2 to 20 bioreactors connected in series, while the number thereof is selected for conditions of the process and the substrate being processed. Furthermore, the bioreactors in series are connected by pipelines, provided with ascending vessels.

Preferably, the digestate valves located at the ends of the sections make possible a violent expansion of the digestate in order to reduce the total number of microbes, which enables a partial sanitation of the effluent.

The system according to the invention does not require the use of large-volume tanks, as the optimal conditions for consecutively proceeding biochemical reactions along with continual temperature and pressure control are thereby provided..

Owing to modular design, the system can be expanded by connecting any number of bioreactors, one to another, while the number thereof is selected for reaction conditions and the type of organic matter being processed.

The method and the system make possible to run the process under elevated pulsating pressure, ensuring the reciprocating substrate movement and mixing the reaction substrate without using mechanical agitators.

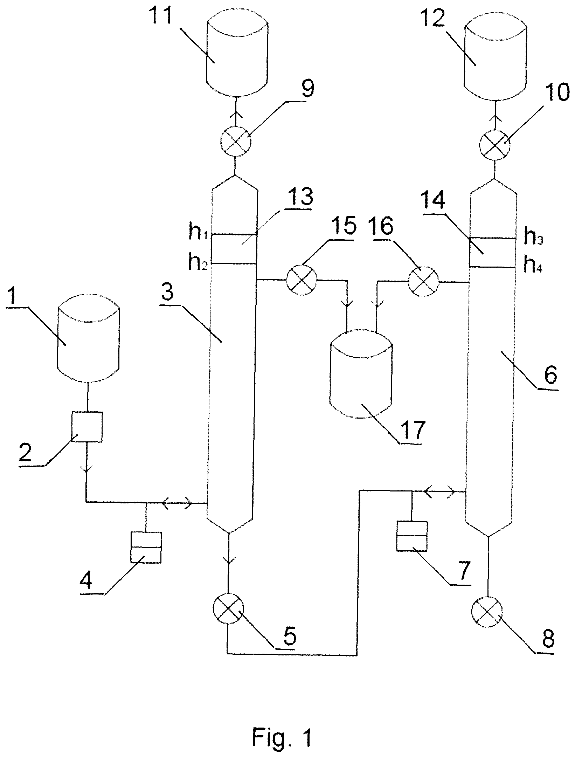

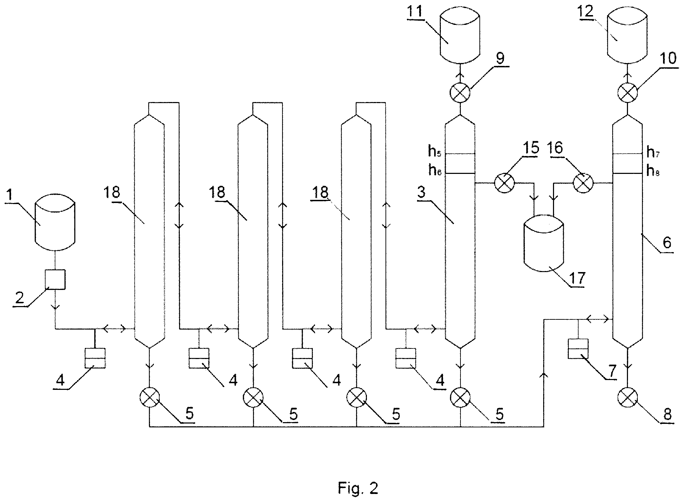

The object of the invention has been explained in embodiments and in drawings, where in Fig. 1 presented is a system comprising one bioreactor in each processing section, and in Fig. 2 presented is a system comprising in the first section 4 bioreactors connected in series.

### Embodiment 1

The wastes subject to processing in a system presented in Fig. 1 consist of sewage sludge mixed with food industry wastes such as whey, dairy effluents, slop, slaughter wastes, poultry litter. The biodegradable material containing organic matter such as proteins, carbohydrates, hydrocarbons and inorganic compounds, wherein the C:N:P ratio is close to 100:2.5:0.5, and the amount of traces elements such as iron, cobalt, zinc and nickel is not lower than 1 mg FeCl₂, 0.1 mg CoCl₂, 0.1 mg NiCl2, 0.1 mg ZnCl₂, and alkalinity corresponds to 1500-3000 mg/l CaCO₃, is fed to a substrate tank **1.** Amount of organic carbon in the substrate, expressed as COD mg/l (chemical oxygen demand), is 5000.

The substrate is supplied by the pump **2** under 8 bar pressure to the first pipe bioreactor **3.** The substrate is fed into the bottom part of the bioreactor **3,** of dimensions: length - 25 m, diameter - 0.1 m, volume - 0.196 m³, where at pH 6.6 - 7.8 and at temperature ca. 39 °C the anaerobic decomposition of the substrate begins. The bioreactor is fed with the substrate in such a manner, that the resultant flow rate could reach 8.6 cm/h. The bioreactor **3** is filled with a bed designed for development of a biofilm that makes possible to catalyse biochemical reactions. The reaction matter in the bioreactor **3** is subject to mixing through a pulsating reciprocating stream of the reaction mixture, caused by pressure changes that are compensated by the ascending vessel **4.** The processing in the bioreactor **3** is run for fifteen days at maximum pressure ca. 8 bar and then through a valve **5,**located in the conical bottom of the bioreactor **3,** 20% of the substrate volume is removed in pulsating manner by the pump **2,** thus carrying away the sedimenting fraction that requires longer digestion time to the second bioreactor **6.** The bioreactor **6** is filled with a bed designed for development of a biofilm able to catalyse biochemical reactions. The pulsating movement of the reaction mixture through the valve **5** causes a reverse motion of reagents in the bioreactor **3** and a advancing movement in the bioreactor **6.** Hydraulic retention time in the bioreactor **6** is fourfold longer than in the reactor **3** and the decomposition process takes ca. 60 days. Technical parameters of both reactors are identical except that the pressure in the bioreactor **6** is by 2 bar lower, which means it is about 6 bar. The reciprocating movement of reagents in the bioreactors is obtained owing to the ascending vessels **4** and **7.** Inorganic constituents are removed at the bottom part of the bioreactor **6** through a check valve **8.** During the process, in different bioreactor zones stabilised are consortia of microorganisms that most effectively carry out predominant biochemical reactions. Stabilisation of the microorganism consortia in the individual bioreactor zones is obtained with the contribution of the biofilm developed on the bioreactor inside walls, where the biofilm surface to volume ratio is 4 m²/m³.

Obtained gaseous products are separated from digestate in upper parts of the bioreactors **3** and **6,** and carried away through pressure gas valves **9** and **10** to a high-pressure gas tank **11** under 8 bar pressure and to a gas tank **12** under 6 bar pressure. The utility biogas is received directly from those tanks.

The gas check valve **9** opens when the gas pressure in the reactor **3** reaches the 8 bar level, while the valve **10** opens when the gas pressure in the reactor **6** reaches the 6 bar level . The sensors **13** and **14** of the substrate level in the bioreactors control the operation of the digestate valves **15** and **16,** owing to which the substrate table levels are maintained in the range from h1 to h2 and from h3 to h4.

The digestate is removed in cyclical manner thus ensuring a quick change of liquid column from the maximum level (h₁ and h₃) to the minimum level (h₂ and h₄) through the digestate valves **15** and **16** into the digestate tank **17.** Quick reduction of the substrate column in the bioreactor **3** and **6** effects in the pressure change within the entire bioreactor, and in pulsating flow that causes the substrate volume changes in the ascending vessels **4** and **7.** Violent digestate expansion effects in partial sanitation thereof.

Owing to atmospheric pressure in the tank **17,** CO₂ degassing is possible. The valve **15** opens when the substrate level in the reactor **3** reaches the value h₁ and closes when this level drops to h₂. The valve **16** opens when the substrate level in the reactor **6** reaches h₃ value, and closes when this level drops to h₄.

As a result, a COD reduction to the value of 92% is obtained and the methane content in the received biogas is higher as compared to the carbon dioxide content.

### Embodiment 2

Shredded plants, rotten or stale food products and animal feed, containing proteins, carbohydrates hydrocarbons, acids, alcohols, aldehydes are subject to processing in a system shown in Fig.2. The biodegradable material wherein the C:N:P ratio is close to 100:2.5:0.5, and the alkalinity corresponds to 2000-2500 mg/l CaCO₃, is fed to a substrate tank **1.** Amount of organic carbon in the substrate, expressed as COD mg/l (chemical oxygen demand), is 4000.

The substrate is supplied continually by the pump **2** under 9 bar pressure to the first bioreactor **18.** The substrate is fed into the bottom chamber of the reactor, of dimensions: height - 20 dm, diameter - 4 dm, volume - ca. 250 dm³, where at pH 6.6 - 7.5 and at temperature 30 °C the anaerobic decomposition of the substrate begins. The reaction mixture is transferred through the next two bioreactors **18** to the bottom part of the last in series bioreactor **3.** In the bioreactors **18** and **3**, each of them filled with a bed where has developed a biofilm able to catalyse biochemical reactions, proceed next stages of anaerobic process, leading to the biogas production and the substrate thickening. The biogas produced in bioreactors **18** and **3** is moved to the upper part of the bioreactor **3.** When the biogas pressure in the upper part of the bioreactor **3** exceeds 9 bar, the valve **9** opens and the biogas enters into the gas tank **11** which is under elevated pressure, lower than 9 bar. The bioreactors 18 are filled with reagents.

The reaction mixture table level in the bioreactor **3** is maintained between **h₅, h₆.** When the substrate level in the reactor reaches the value **h₅** the valve **15** opens and a part of reacted mixture enters the digestate tank **19.** This action stops when the level of the reaction mixture in the bioreactor **3** has dropped to the level **h₆.** The quick lowering the level of reagents between values **h₅, h₆,** effects in pressure drop in reactors **18** and **3.** At the same time, a part of the mixture accumulated in ascending vessels **4** is transferred to bioreactors. And then the direction of movement corresponds to the conducted process. The movement in opposite direction is caused by rising pressure of the biogas that collects in the upper part of the bioreactor **3,** which leads to changing the level of the reaction mixture, the mixture being received into the ascending vessels. Cyclical small pressure changes ensure the substrate mixing in each of bioreactors **18** and **3** through a pulsating reciprocating stream of the reaction mixture as a result of the mixture movement between bioreactors and ascending vessels.

The collected sediment, requiring longer digestion time, is removed through the valves **5** placed in a conical bottom of each bioreactor **18** and transferred to the bioreactor **6,** of the following dimensions: height - 30 dm, diameter - 4 dm, volume - ca. 380 dm³. The process in the bioreactor **6** proceeds under elevated hydraulic pressure, changing in pulses from 6 to 7 bar, with participation of microorganisms. When the biogas pressure in the upper part of the bioreactor **6** exceeds 7 bar, the valve **10** opens and the biogas is removed into the gas tank **12** which is under elevated pressure, ca. 7 bar.

The table level of liquid phase in the bioreactor **6** is maintained between **h₇** and **h₈.** When the substrate level in the reactor reaches the value **h₇** the valve **16** opens and a part of reacted mixture is removed to the digestate tank **17.** This action stops when the level of the substrate in the bioreactor **6** has dropped to the level **h₈.** The quick lowering the level of substrate between values **h₇** and **h₈** effects in pressure drop in the reactor **6.** At the same time, a part of the liquid phase accumulated in the ascending vessel **7** is transferred to the bioreactor **6.** And then the direction of movement corresponds to the conducted process. When the pressure in the bioreactor increases to 7 bar as a result of the biogas flowing out, the opposite process begins, where the reaction mixture, flowing in the opposite direction, is received into the ascending vessel.

The fraction received from the bioreactor **6** through the valve **8** contains mostly a mixture of indecomposable solid substances such as sand.

As a result, a COD reduction to the value of 90% is obtained and the methane content in the received biogas is higher as compared to the carbon dioxide content.

### List of designations in the drawings

- 1: - substrate tank
- 2: - substrate pump
- 3: - bioreactor for quick-decomposing fraction
- 4: - ascending vessel
- 5: - transfer valve
- 6: - bioreactor for slow-decomposing fraction
- 7: - ascending vessel for bioreactor for slow-decomposing fraction
- 8: - check valve
- 9: - gas valve
- 10: - gas valve
- 11: - high-pressure gas tank
- 12: - gas tank
- 13: - level sensor
- 14: - level sensor
- 15: - digestate valve
- 16: - digestate valve
- 17: - digestate tank
- 18: - additional bioreactors for quick-decomposing fraction

## Claims

1. A method of processing organic matter by anaerobic digestion, with biogas production, the said method comprising the process stages of hydrolysis of macromolecular compounds to smaller molecules, (ii) transformation of those to organic acids followed by (iii) processing to acetates, methanol and carbon dioxide and finally (iv) processing to methane, wherein each of the Z process stages (i)-to-(iv) proceeds in spontaneously formed separate reaction zones with participation of microorganisms specific for the giving stage under elevated hydraulic pressure changing in a pulsating manner within the range from atmospheric pressure to 500 bar, and at the temperature ranging from 4°C to 180°C, wherein each of the consecutive process stages (i)-to-(iv) proceeds in a bioreactor chamber of length suitable for the reactions proceeding therein, whereas the microorganisms appropriate for the process that proceeds in a given zone are immobilized in a bed filled in each reaction zone, the sequence of process stages (i)-to-(iv) follows in a chamber of at least one bioreactor, with mixing of reagents in each zone, the reaction products are transferred from one zone to another in form of a pulsating reciprocating stream of reaction mixture at the rate from 0.01 m/h to 180 m/h, and at the same time, the obtained biogas and liquid decomposition products are carried away, while the undissolved fraction that needs longer time of digestion is constantly separated and-transferred to a bioreactor of extended hydraulic retention time which is operated in parallel.

2. The method according to the claim 1, wherein the said separated fraction requiring longer digestion time is subject to breaking up by mixing with unreacted inorganic molecules, such as sand.

3. The method according to the claim 1, wherein each process stage is run at a selected pH, which ranges between 4.5- 8.0 for the stage of macromolecular compounds hydrolysis and for the stage of conversion thereof into organic acids, pH range is 5-7.5 in the stage of conversion thereof to acetates, methanol and carbon dioxide, and in the stage of processing to methane the pH range is 6 - 9.

4. The method according to the claim 1, wherein the said received biogas is stored under pressure created during the process.

5. The method according to the claim 1, wherein the said compressed biogas is used for pulsating agitation in the bioreactor.

6. The method according to the claim 1, wherein temperature, density and pressure is controlled in each of said reaction zones.

7. A system for processing organic matter by anaerobic digestion, comprising a bioreactor filled with a bed enabling development of a biofilm of microorganisms, wherein the said system comprises two sections operating under different pressures, a first section for processing a quick-decomposing fraction and a second section for processing a slow decomposing fraction, the first section comprises connected to one another consecutively: a substrate tank (1), a substrate metering pump (2), an ascending vessel (4) providing reciprocating movement of the substrate throughout reaction zones in the section and at least one bioreactor (3), to process the quick decomposing fraction in reaction zones spontaneously formed therein, -wherein bioreactor (3), comprises a separatory zone which is situated in its upper part is connected by a pipeline provided with a digestate valve (15) to the digestate tank (17), for removing from the said bioreactor (3) the digestate that is separated from the biogas, which biogas is received from the separatory zone of the said bioreactor (3) by a pipeline provided with a gas valve (9), and an outlet pipeline which is mounted in the bottom of the bioreactor and is provided with a valve (5) for removing the sedimenting, slow-decomposing fraction and transferring it to feed a bioreactor (6) comprised in the second section, the second section comprises an ascending vessel (7) ensuring reciprocating movement of the substrate and at least one bioreactor (6) to process the slow decomposing fraction, wherein bioreactor (6) comprises a separatory zone, which is situated in its upper part and connected by a pipeline provided with a digestate valve (16) to the digestate tank (17) for removing from the said bioreactor (6) the digestate that is separate from the biogas, which biogas is received from the separatory zone of the said bioreactor (6) by a pipeline provided with a gas valve (10), and a check valve (8) which is connected at its bottom part.

8. The system according to claim 7, wherein the said system is provided with digestate valves (15) and (16) and check valves (8), connected to the said bioreactors.

9. The system according to claim 7, wherein both said sections are connected to the biogas pressure tanks (11) and (12), where the biogas is stored under pressure.

10. The system according to claim 7, wherein the section for processing the quick-decomposing fraction comprises from 2 to 20 bioreactors (18) and .(3), connected in series, and the number of said bioreactors is selected taking into account the conditions of the process and the substrate being processed.

11. The system according to claim 10, wherein from among several bioreactors connected in series only the last bioreactor from the series (3) is connected to the biogas receiving tank .

12. The system according to claim 7, wherein the bottom of each said bioreactor is of conical shape and each said bioreactor is filled with a bed immobilising the microorganisms specific for a given stage and conditions of running the anaerobic digestion.

13. The system according to claim 10, wherein the all said bioreactors connected in series in the section for processing the quick-decomposing fraction, are connected by pipelines provided with ascending vessels (5).

## Patentansprüche

1. Verfahren zur Verarbeitung von organischem Material durch anaerobe Vergärung mit Biogasproduktion; der Prozess umfasst die Schritte (i) der Hydrolyse makromolekularer Verbindungen zu kleineren Molekülen, (ii) der Umwandlung in organische Säuren, gefolgt von (iii) der Umwandlung in Acetate, Methanol und Kohlendioxid und schließlich (iv) der Umwandlung in Methan, jede der Verfahrensstufen (i) bis (iv) findet in spontan geschaffenen separaten Reaktionszonen unter Beteiligung stufenspezifischer Mikroorganismen in einem erhöhten hydraulischen System statt, der Druck variiert pulsierend von Atmosphärendruck bis 500 bar und bei einer Temperatur von 4°C bis 180°C, jede der nachfolgenden Verfahrensstufen (i) bis (iv) findet in einer Bioreaktorkammer von angemessener Länge für die dort stattfindenden Reaktionen statt, während die für das in einer bestimmten Zone ablaufende Verfahren geeigneten Mikroorganismen in einem in jeder Reaktionszone gefüllten Bett immobilisiert; werden, die Abfolge der Verfahrensschritte (i) bis (iv) in einer Kammer! mindestens eines Bioreaktors erfolgt, wobei in jeder Zone Reagenzien gemischt" werden, die Reaktionsprodukte in Hin- und Herpulsbewegung der Mischung mit einer Geschwindigkeit von 0,01 m / h bis 180 m / h von einer Zone in eine andere weitergeleitet werden, während das entstehende Biogas und die flüssigen Zersetzungsprodukte abgeführt werden, während die ungelöste Fraktion, die eine längere Verdauungszeit benötigt, kontinuierlich abgetrennt und in einen parallel arbeitenden Bioreaktor mit verlängerter hydraulischer Verweilzeit weitergeleitet wird.

2. Verfahren nach Anspruch 1 **dadurch gekennzeichnet, daß** der die genannte abgetrennte Fraktion, die eine längere Verdauungszeit benötigt, durch Mischen mit nicht umgesetzten anorganischen Partikeln wie Sand zersetzt wird.

3. Verfahren nach Anspruch 1 **dadurch gekennzeichnet, daß** der jede Stufe des Verfahrens bei einem gewählten pH-Wert zwischen 4,5 und 8,0 für die Stufe der makromolekularen Hydrolyse und für die Stufe der Umwandlung in organische Säuren durchgeführt wird, der pH-Bereich beträgt 5 bis 7,5 für die Stufe der Umwandlung von Acetat, Methanol und Kohlendioxid und 6 bis 9 für die Stufe der Umwandlung von Methan.

4. Verfahren nach Anspruch 1 **dadurch gekennzeichnet, daß** der das bereits erwähnte, gewonnene Biogas unter Prozessdruck gespeichert wird.

5. Verfahren nach Anspruch 1 **dadurch gekennzeichnet, daß** der das besagte komprimierte Biogas zur pulsierenden Mischung in einem Bioreaktor verwendet wird.

6. Verfahren nach Anspruch 1 **dadurch gekennzeichnet, daß** der Temperatur, Dichte und Druck in jeder dieser Reaktionszonen kontrolliert werden.

7. System zur Verarbeitung von organischem Material durch anaerobe Gärung, einschließlich eines bettgefüllten Bioreaktors zur Entwicklung eines mikrobiellen Biofilms, wobei das genannte System aus zwei bei unterschiedlichem Druck arbeitenden Abschnitten besteht, einem ersten Abschnitt zur Behandlung der sich schnell zersetzenden Fraktion und einem zweiten Abschnitt zur Behandlung der sich langsam zersetzenden Fraktion, wobei der erste Abschnitt wiederum folgende miteinander verbundene Teile hat: einen Substratbehälter (1), eine Substratdosierpumpe (2), ein Ausdehnungsgefäß (4) zur Gewährleistung der Hin- und Herbewegung des Substrats in den Reaktionszonen des Abschnitts und mindestens einen Bioreaktor (3) zur Verarbeitung der Schnellzersetzungsfraktion in spontan darin gebildeten Reaktionszonen, wobei der Bioreaktor (3) eine Trennzone enthält, die sich in seinem oberen Teil befindet, durch eine mit einem Gärrestventil (15) ausgestattete Rohrleitung mit dem Gärrestbehälter (17) verbunden ist, um aus dem genannten Bioreaktor (3) den Gärrest abzutrennen, der von dem Biogas abgetrennt wird, das aus der Trennzone des genannten Bioreaktors (3) entnommen wird, und zwar mittels einer mit einem Gasventil (9) ausgestatteten Rohrleitung und einer am Boden des Bioreaktors angebrachten Auslassrohrleitung, die mit einem Ventil (5) zur Entfernung der sich ablagernden, langsam zersetzenden Fraktion und mit einem Bioreaktor zu derer Weiterleitung ausgestattet ist (6), der sich im zweiten Abschnitt befindet, wobei der zweite Abschnitt ein Ausdehnungsgefäß (7) zur Gewährleistung der Hin- und Herbewegung des Substrats und mindestens einen Bioreaktor (6) zur Verarbeitung der sich langsam zersetzenden Fraktion enthält, wobei der Bioreaktor (6) eine Trennzone enthält, die sich im oberen Teil befindet und durch eine mit einem Gärrestventil (16) und einem Gärrestbehälter (17) ausgestattete Rohrleitung verbunden ist, um aus dem genannten Bioreaktor (6) das von dem Biogas abgetrennte Gärrest abzuführen, das Biogas aus der Trennzone des genannten Bioreaktors (6) erhält, wobei das Biogas aus der Trennzone des genannten Bioreaktors (6) durch eine mit einem Gasventil (10) und einem Inspektionsventil (8) ausgestattete Rohrleitung, die im unteren Teil angeschlossen ist, zugeführt wird.

8. System nach Anspruch 7 **dadurch gekennzeichnet, daß** genannte System mit Gärrestventilen (15) und (16) und Inspektionsventilen (8) ausgestattet ist, die mit den genannten Bioreaktoren verbunden sind.

9. System nach Anspruch 7 **dadurch gekennzeichnet, daß** beide Abschnitte mit den Biogasdruckbehältern (11) und (12) verbunden sind, in denen das Biogas unter Druck gespeichert wird.

10. System nach Anspruch 7 **dadurch gekennzeichnet, daß** der Verarbeitungsabschnitt für die sich schnell zersetzende Fraktion 2 bis 20 in Reihe geschaltete Bioreaktoren (18) und (3) enthält und die Anzahl dieser Bioreaktoren unter Berücksichtigung der Prozessbedingungen und des zu verarbeitenden Substrats gewählt wird.

11. System nach Anspruch 7 **dadurch gekennzeichnet, daß** von den mehreren in Reihe geschalteten Bioreaktoren nur der letzte in Reihe verbundene Bioreaktor (3) an den Biogasaufnahmebehälter angeschlossen ist.

12. System nach Anspruch 7 **dadurch gekennzeichnet, daß** der Boden jedes Bioreaktors konisch geformt ist und jeder Bioreaktor mit einem Bett gefüllt ist, die Mikroorganismen immobilisiert, die für das Stadium und die Bedingungen der anaeroben Gärung spezifisch sind.

13. System nach Anspruch 7 **dadurch gekennzeichnet, daß** alle diese Bioreaktoren in dem Abschnitt für die Verarbeitung der sich schnell zersetzenden Fraktion durch Rohrleitungen, die mit Ausdehnungsgefäßen ausgestattet sind, in Reihe geschaltet sind (5).

## Revendications

1. Méthode de traitement de la matière organique par digestion anaérobie avec production de biogaz comprenant les étapes de traitement suivantes : (i) hydrolyse des composés macromoléculaires en molécules plus petites, (ii) transformation de celles-ci en acides organiques, suivie de (iii) transformation en acétates, méthanol et dioxyde de carbone, et finalement (iv) transformation en méthane ; chacune des étapes (i) à (iv) se déroule dans des zones de réaction distinctes formées spontanément, en présence de micro-organismes spécifiques à l'étape en question, à pression hydraulique élevée variant par pulsions dans une plage de pression atmosphérique allant jusque 500 bars, et à une température comprise entre 4°C et 180°C ; chacune des étapes (i) à (iv) se déroule dans une chambre de bioréacteur de longueur adaptée aux réactions qui s'y produisent, et les micro-organismes propres au processus qui se déroule dans chaque zone de réaction sont chaque fois immobilisés dans un lit ; la suite des étapes (i) à (iv) du processus se déroule dans une chambre d'au moins un bioréacteur, avec mélange des réactifs dans chaque zone; les produits de la réaction passent d'une zone à l'autre sous la forme d'un flux pulsé, en va-et-vient, à une vitesse de 0,01 m/h à 180 m/h ; le biogaz et les produits de décomposition liquides obtenus sont évacués, tandis que la fraction non dissoute nécessitant un temps de digestion plus long est régulièrement soustraite et transvasée dans un bioréacteur à temps de rétention hydraulique prolongé fonctionnant en parallèle.

2. Méthode selon la revendication 1, où la fraction séparée demandant un temps de digestion plus long est fractionnée par mélange avec des molécules inorganiques non réactives, telles que le sable.

3. Méthode selon la revendication 1, où chaque étape du processus est effectuée à un pH sélectionné compris entre 4,5 et 8,0 pour l'étape d'hydrolyse des composés macromoléculaires et l'étape de conversion de ces derniers en acides organiques, entre 5 et 7,5 pour l'étape de conversion de ces derniers en acétates, méthanol et dioxyde de carbone, et entre 6 et 9 pour l'étape de transformation en méthane.

4. Méthode selon la revendication 1, où le biogaz recueilli est stocké sous l'effet de la pression créée pendant le processus.

5. Méthode selon la revendication 1, où le biogaz sous pression est utilisé pour agiter par pulsions les matières dans le bioréacteur.

6. Méthode selon la revendication 1, où la température, la densité et la pression sont contrôlées dans chacune des zones de réaction.

7. Système de traitement de la matière organique par digestion anaérobie, comprenant un bioréacteur rempli d'un lit permettant la formation d'un biofilm de microorganismes ; le système comprend deux sections fonctionnant à des pressions différentes, la première section servant au traitement de la fraction à décomposition rapide, et la seconde, au traitement de la fraction à décomposition lente ; la première section se compose d'un ensemble de sections reliées entre elles, comme suit: un réservoir de substrat (1), une pompe de dosage de substrat (2), un vase d'expansion (4) assurant le mouvement de va-et-vient du substrat dans les zones de réaction de la section, et au moins un bioréacteur (3) destiné à traiter la fraction à décomposition rapide dans les différentes zones de réaction qui s'y forment spontanément ; le bioréacteur (3), comprend une zone de séparation située dans sa partie supérieure et reliée par une conduite équipée d'une vanne de digestat (15) à un réservoir de digestat (17), afin d'évacuer du bioréacteur (3) le digestat séparé du biogaz ; le biogaz est recueilli dans la zone de séparation du bioréacteur (3) par une conduite munie d'une vanne de gaz (9) ; une conduite de sortie installée au fond du bioréacteur et munie d'une vanne (5) évacue la fraction sédimentaire à décomposition lente et l'achemine vers le bioréacteur (6) de la deuxième section ; la deuxième section comprend un vase d'expansion (7) assurant le mouvement de va-et-vient du substrat et au moins un bioréacteur (6) pour traiter la fraction à décomposition lente ; le bioréacteur (6) comprend une zone de séparation située dans sa partie supérieure et reliée par une conduite munie d'une vanne de digestat (16) à un réservoir de digestat (17), pour évacuer du bioréacteur (6) le digestat séparé du biogaz ; ce dernier est recueilli dans la zone de séparation du bioréacteur (6) par une conduite munie d'une vanne de gaz (10), et une vanne anti-retour (8) est reliée à sa partie inférieure.

8. Système selon la revendication 7, dans lequel ce système est pourvu de vannes de digestat (15) et (16) et de vannes anti-retour (8) reliées aux bioréacteurs.

9. Système selon la revendication 7, dans lequel les deux sections sont reliées à des réservoirs de biogaz sous pression (11) et (12), où le biogaz est stocké sous pression.

10. Système selon la revendication 7, dans lequel la section de traitement de la fraction à décomposition rapide comprend de 2 à 20 bioréacteurs (18) et (3) reliés en série, le nombre de bioréacteurs étant choisi en fonction des conditions du processus et du substrat à traiter.

11. Système selon la revendication 10, dans lequel seul le dernier bioréacteur (3) d'une série pouvant en comporter plusieurs connectés l'un à l'autre est relié au réservoir de récupération du biogaz.

12. Système selon la revendication 7, dans lequel le fond de chacun des bioréacteurs est de forme conique, et chacun des bioréacteurs est rempli d'un lit immobilisant les micro-organismes spécifiques à l'étape concernée et aux conditions de déroulement de la digestion anaérobie.

13. Système selon la revendication 10, dans lequel tous les bioréacteurs reliés en série dans la section de traitement de la fraction à décomposition rapide sont reliés par des conduites munies de vases d'expansion (5).
